# EUROPEAN PATENT SPECIFICATION

(11) **EP 4 106 555 B1**
(45) Date of publication and mention of the grant of the patent: **07.02.2024**
(21) Application number: 21705952.6
(22) Date of filing: 18.02.2021
(51) Int. Cl.: A24B 3/14, A24B 15/12, A24B 15/16, A24B 15/24

(54) **HIGH-DENSITY MULTILAYER RECONSTITUTED PLANT SHEET**
HOCHDICHTES MEHRLAGIGES REKONSTITUIERTES PFLANZENBLATT
FEUILLE VÉGÉTALE RECONSTITUÉE MULTICOUCHE À HAUTE DENSITÉ

(30) Priority: 18.02.2020 FR 2001614
(43) Date of publication of application: 28.12.2022
(73) Proprietor: SWM Luxembourg, 5326 Contern (LU)
(72) Inventor: RIGOULAY, Christophe, 72700 SPAY (FR); JARDIN, Cédric, 72700 SPAY (FR)
(74) Representative: Plasseraud IP
(86) International application number: PCT/EP2021/053976
(87) International publication number: WO 2021/165372

(56) References cited:
- WO-A1-2019/068930
- WO-A1-2020/025712
- FR-A1- 3 070 237
- US-A- 3 870 054

## Description

### Technical field

The present disclosure belongs to the field of devices for heating tobacco without burning it and has as its subject a high-density reconstituted plant sheet obtained by a papermaking process.

### Context of the invention

A large number of devices for heating tobacco without burning it have been developed in order to avoid the formation of the harmful constituents during tobacco combustion. By way of example, mention may be made of the applications published under numbers WO 2016/026810 and WO 2016/207407 which describe such devices. Tobacco-filled sticks are placed into these devices, the tobacco is then heated by the device at a temperature below the combustion temperature without being burnt, which leads to the formation of an aerosol when the user sucks in air through the device. The aerosol generated during the heating of the tobacco replaces the cigarette smoke, and has advantageous organoleptic properties when it is inhaled by the user. This allows the user to inhale nicotine and the tobacco aromas while at the same time very significantly reducing said user's exposure to the harmful constituents.

In order for the user to adopt these heating devices, it is important for the experience obtained with said devices to be as close as possible to the experience obtained with a conventional cigarette, that is to say satisfactory organoleptic properties for each puff.

Natural tobacco leaves may not be suitable for such devices since they do not make it possible for the user to obtain a satisfactory experience, in particular they do not make it possible to easily generate a sufficient amount of aerosol.

Reconstituted tobacco is more suitable for these heating devices, since it makes it possible to easily generate a significant amount of aerosol. However, the tobacco aromas in the aerosol may be diluted, such that it is possible to further improve the organoleptic properties of the aerosols formed from the reconstituted tobacco. Document WO2019/068930 discloses such a reconstituted plant sheet.

One way of improvements consists in increasing the mass of reconstituted tobacco present in the stick in order to generate aerosols more loaded with tobacco aromas and hence having satisfactory organoleptic properties. However, this can lead to an increase in the draw resistance, which can prevent the user from being able to draw puffs in a satisfying manner.

There is therefore a compromise to be found between the draw resistance and the organoleptic properties of an aerosol.

### Summary

It is thus to the credit of the inventors to have found that it was possible to meet this need by means of a reconstituted plant sheet comprising:
- two fibrous supports, each fibrous support comprising refined plant fibers and a plant extract,
- a plant extract composition between the two fibrous supports, and
- an aerosol-generating agent,
characterized in that the density of the reconstituted plant sheet is greater than or equal to 0.60 g/cm³.

Compared to a conventional reconstituted plant sheet, i.e. of lower density and comprising a single fibrous support, the reconstituted plant sheet according to the invention makes it advantageously possible to increase the mass of tobacco introduced into the heating device so that the aerosol formed has improved organoleptic properties and so that the user's satisfaction is not degraded by an excessively high draw resistance. The reconstituted plant sheet according to the invention thus makes it possible to obtain an advantageous compromise between the draw resistance and the enhanced organoleptic properties of the aerosols, which is not possible with the conventional reconstituted plant sheet.

A second subject of the invention is a process for producing a reconstituted plant sheet according to the invention, comprising the following steps:
a) passing the refined plant fibers through a papermaking machine so as to produce a base web,
b) bringing a plant extract into contact on the base web to obtain a fibrous support, and
c) bringing a plant extract composition into contact between two fibrous supports so as to obtain the wet reconstituted plant sheet, and
d) drying the wet reconstituted plant sheet to produce the reconstituted plant sheet,
wherein an aerosol-generating agent is incorporated during step b) and/or during step c).

A third subject of the invention is a consumable for a device for heating tobacco without burning it, comprising a reconstituted plant sheet according to the invention.

A fourth subject of the invention is the use of a reconstituted plant sheet according to the invention or of a consumable according to the invention in a heating device, in particular a device for heating tobacco without burning it.

### Description of embodiments

According to a first subject, the invention relates to a reconstituted plant sheet comprising:
- two fibrous supports, each fibrous support comprising refined plant fibers and a plant extract,
- a plant extract composition between the two fibrous supports, and
- an aerosol-generating agent,
characterized in that the density of the reconstituted plant sheet is greater than or equal to 0.60 g/cm³.

Typically, the density of the reconstituted plant sheet may be between 0.62 g/cm³ and 1.50 g/cm³, in particular between 0.65 g/cm³ and 1.25 g/cm³, very particularly between 0.70 g/cm³ and 1 g/cm³, even more particularly between 0.71 g/cm³ and 0.89 g/cm³.

The density of the reconstituted plant sheet is calculated by dividing its basis weight by its thickness.

To determine the basis weight of the plant sheet, the following method may be used:
a sample of 0.25 m² is cut out with a template (dimensions: 57.5 x 43.5 cm) at approximately 15 cm from the edge of the reconstituted plant sheet to be analyzed. The sample is then folded in four and placed on a hotplate so as to be dried thereon in order to remove the water without removing the aerosol-generating agent.
The dried sample is then weighed to determine the basis weight of the plant sheet.

To determine the thickness of the plant sheet, the method described in standard NF EN ISO 534 (December 2011) suitable for the reconstituted plant sheets may be used:
- the average thickness of the control parchment paper used to measure the thickness of the reconstituted plant sheet is measured (minimum of 6 measurements on a layer, on sites pinpointed on the paper),
- the reconstituted plant sheet sample is placed between 2 thicknesses of parchment paper,
- as soon as the probe of the micrometer has been put in place, there is a waiting period of 30 seconds before taking the measurement (stabilization of the sample for thickness measurement),
- a minimum of 6 measurements are taken at the sites pinpointed on the sheet of parchment paper,
- the calculated thickness of the reconstituted plant sheet is the average of the overall thickness measured (reconstituted plant sheet + 2 sheets of parchment paper) from which is subtracted 2 times the average thickness of the parchment paper.

Typically, the reconstituted plant sheet may have a basis weight of greater than 200 g/m², in particular between 275 g/m² and 950 g/m², very particularly between 300 g/m² and 650 g/m², even more particularly between 310 g/m² and 460 g/m².

Typically, the reconstituted plant sheet may have a thickness of between 250 µm and 1050 µm, in particular between 350 µm and 800 µm, very particularly between 360 µm and 700 µm, even more particularly between 375 µm and 515 µm.

According to one particular embodiment, the reconstituted plant sheet may have a density of between 0.71 g/cm³ and 0.89 g/cm³, a basis weight of between 310 g/m² and 460 g/m² and a thickness of between 375 µm and 515 µm.

Those skilled in the art will know how to adapt the basis weight and the thickness of the reconstituted plant sheet in order to achieve the desired high density. For example, to obtain these thickness and/or density values, the reconstituted plant sheet of the invention may undergo a calendering step. Thus, according to one embodiment of the invention, the reconstituted plant sheet of the invention may be calendered.

For the purposes of the present application, "fibrous support" denotes a base web made of refined fibers of the plant and comprising a plant extract. The base web is typically obtained by a papermaking process.

Typically, the fibrous support may have a basis weight of between 60 g/m² and 200 g/m², in particular between 80 g/m² and 175 g/m², very particularly between 100 g/m² and 155 g/m².

The fibrous support may have a thickness of between 100 µm and 300 µm, in particular between 150 µm and 275 µm, very particularly between 180 µm and 265 µm.

The base web may have a basis weight of between 25 g/m² and 150 g/m², in particular the minimum value for the basis weight may be 55 g/m², 60 g/m², 65 g/m², 70 g/m², 75 g/m², 80 g/m², or 85 g/m².

The base web has, for example, a thickness of between 70 µm and 300 µm, in particular between 140 µm and 275 µm, very particularly between 170 µm and 250 µm.

Advantageously, a base web having a basis weight and/or a thickness comprised within the above ranges makes it possible to obtain a reconstituted plant sheet presenting the desired high density.

The basis weight and the thickness of the base web and of the fibrous support are typically measured by the same methods as the basis weight and the thickness of the reconstituted plant sheet described above.

According to one particular embodiment, the reconstituted plant sheet may comprise more than two fibrous supports, in particular 3, 4 or 5 fibrous supports, more particularly 3 or 4 fibrous supports.

According to this particular embodiment, a plant extract composition may be included between two fibrous supports.

For the purposes of the present application, the term "refined fibers of the plant" denotes fibers of the plant which have undergone a refining step enabling fibrillation and/or cutting of the fibers of the plant. The refining step is conventionally carried out in a papermaking process, such as the papermaking process producing reconstituted papermaking tobacco. However, the refining step is not carried out in a process producing cast leaf reconstituted tobacco.

Typically, the fibrous support may comprise refined fibers of one and the same plant or of several plants.

For the purposes of the present application, the term "plant extract" denotes all of the water-soluble products of the plant. Advantageously, the plant extract comprises nicotine, and compounds conferring organoleptic properties and/or therapeutic properties to the aerosol.

For the purposes of the present application, the term "plant extract composition" denotes a composition comprising the plant extract as defined above.

The plant extract composition forms a layer between two fibrous supports of the reconstituted plant sheet of the invention. Advantageously, the plant extract composition makes it possible to maintain the cohesion of the reconstituted plant sheet of the invention by keeping the two fibrous supports together, for example by sticking one to the other, without using adhesive exogenous to the plant.

For example, the concentration of solids of plant extract in the plant extract composition may be greater than 45%, in particular between 50% and 70%, very particularly between 55% and 65%, even more particularly between 58% and 62%.

Advantageously, the plant extract composition having such a concentration of solids of plant extract promotes the cohesion of the reconstituted plant sheet of the invention.

The plant extract composition may comprise, in addition to the plant extract, the aerosol-generating agent, additives or mixtures thereof.

By way of additives, mention may be made of plant dust; a diluent such as CaCO₃; a texturing agent such as guar gum, potato starch, agar-agar or mixtures thereof; a powdered flavoring such as cacao powder, tripotassium citrate, a powdered aromatic preparation or mixtures thereof; an encapsulated flavoring; a colorant such as beta-carotene, powdered apricot juice, turmeric or mixtures thereof; a gel such as a silica gel, a polysaccharide gel, an alumina gel or mixtures thereof; or mixtures thereof, in particular plant dust.

Plant dust may comprise aromatic compounds conferring plant aromas on the aerosol. The plant dust may be derived from various plant parts, the plant parts being parts of the plant themselves or the result of the processing of various plant parts. The plant dust may be obtained by the treatment of one or more plant parts, such as shredding, threshing or mixing and shredding the plant parts.

For the purposes of the present application, the term "aerosol-generating agent" denotes a compound which allows the formation of an aerosol when it is heated, for example in contact with hot air.

The aerosol-generating agent may be present in one of the two fibrous supports, in both fibrous supports, in the plant extract composition or mixtures thereof, in particular in both fibrous supports, in the plant extract composition or in a mixture thereof, in particular in both fibrous supports.

Let S_{AG} be the total content by weight of solids of the aerosol-generating agent included in the reconstituted plant sheet of the present invention. Typically, S_{AG} may be between 10% and 30%, in particular between 12% and 25%, very particularly between 13% and 22%, even more particularly between 15% and 21.5%.

The aerosol generated by a reconstituted plant sheet having an S_{AG} greater than the ranges mentioned above causes unwanted burning of the mouth and/or the throat (phenomenon known as "hot puff").

Typically, the aerosol-generating agent may be a polyol, a non-polyol or a mixture thereof. Typically, an aerosol-generating agent that is a polyol may be sorbitol, glycerol, propylene glycol, triethylene glycol, erythritol, propanediol or mixtures thereof. Typically, an aerosol-generating agent that is a non-polyol may be lactic acid, benzyl benzoate, glyceryl diacetate, glyceryl triacetate, triethyl citrate, isopropyl myristate or mixtures thereof.

According to one embodiment, the aerosol-generating agent is glycerol, propylene glycol, or a mixture of glycerol and propylene glycol, glycerol being preferred.

An aerosol is generated during heating of the reconstituted plant sheet of the invention. Advantageously, the plant extract which comprises aromatic compounds confers aromas from the plant on this aerosol. By simply changing reconstituted plant sheet, the user may easily vary the aromas of the aerosol generated by heating said reconstituted plant sheet.

The organoleptic properties and the therapeutic properties of the aerosol formed by heating said reconstituted plant sheet may depend on the content by weight of solids of the plant extract included in the reconstituted plant sheet of the present invention.

The total content by weight of solids of the plant extract depends on the plant used and, more particularly, on the content of aromatic compounds or compounds having therapeutic properties of the plant used.

Let S_{P} be the total content by weight of solids of the plant extract included in the reconstituted plant sheet of the present invention. Typically, Sₚ may be between 30% and 60%, in particular between 35% and 55%, even more particularly between 37% and 50%.

Advantageously, these high contents make it possible to generate an aerosol having very satisfactory organoleptic properties.

To determine S_{P}, use may be made of the following method:
The reconstituted plant sheet to be analyzed is ground in order to achieve a particle size of less than or equal to 1 mm. The reconstituted plant sheet is then mixed with boiling water for 45 minutes in order to extract all of the plant extract. S_{P} is calculated by the difference between the dry weight of the sample of reconstituted plant sheet to be analyzed and the dry weight of the fibrous residue after extraction.

According to one embodiment, the sum of the total content by weight of solids of plant extract and the total content by weight of solids of aerosol-generating agent, S_{P} + S_{AG}, may be between 40% and 80%, in particular between 45% and 75%, very particularly between 50% and 72%, even more particularly between 55% and 70%.

According to one embodiment, the ratio between the total content by weight of solids of plant extract and the total content by weight of solids of aerosol-generating agent, S_{P}/S_{AG}, is between 1.0 and 3.5, in particular between 1.25 and 3.25, very particularly between 1.50 and 3.10, even more particularly between 1.75 and 2.70.

Advantageously, the organoleptic properties of the aerosol formed are even more satisfactory when the reconstituted plant sheet according to the invention has an S_{P}/S_{AG} ratio in the ranges mentioned above.

According to one particular embodiment, the reconstituted plant sheet have an S_{AG} between 16% and 21.5%, an S_{P} between 37% and 50%, and an S_{P} + S_{AG} comprised between 55% and 70%.

According to one particular embodiment, the reconstituted plant sheet may have a density of between 0.71 g/cm³ and 0.89 g/cm³, a basis weight of between 310 g/m² and 460 g/m² and a thickness of between 375 µm and 515 µm, an S_{AG} of between 16% and 21.5%, an S_{P} of between 37% and 50%, and an S_{P} + S_{AG} of between 55% and 70%.

According to a very particular embodiment, the reconstituted plant sheet may have a density of between 0.83 g/cm³ and 0.89 g/cm³, a basis weight of between 310 g/m² and 460 g/m² and a thickness of between 375 µm and 515 µm, an S_{AG} of between 16% and 20%, an S_{P} of between 43% and 50%, and an S_{P} + S_{AG} of between 60% and 70%.

The plant fibers, the plant extract of the fibrous supports, and the plant extract of the plant extract composition may be independently obtained from a plant chosen from spore-producing plants, seed-producing plants or a mixture thereof. In particular, the plant may be a plant chosen from the tobacco plant, food plants, aromatic plants, fragrant plants, medicinal plants, plants of the family *Cannabaceae,* and mixtures thereof.

According to one particular embodiment, the plant is the tobacco plant.

If the plant is a medicinal plant, the aerosol generated by heating the reconstituted plant sheet may also have therapeutic properties so that the reconstituted plant sheet may be used for a therapeutic treatment.

Advantageously, a plant extract obtained from a plant mixture makes it possible to offer a broad panel of organoleptic properties and/or therapeutic properties. A plant mixture also makes it possible to counteract the unpleasant organoleptic properties of a plant of the mixture, for example a medicinal plant, with the pleasant organoleptic properties of another plant of the mixture, for example the tobacco plant, an aromatic plant or a fragrant plant.

According to one embodiment, the plant fibers may be obtained from a first plant and the plant extract of the fibrous supports and of the plant extract composition may be obtained from a second plant.

According to an alternative embodiment, the plant fibers and the plant extract of the fibrous supports may be obtained from a first plant and the plant extract of the plant extract composition may be obtained from a second plant.

According to another alternative embodiment, the plant fibers and the plant extract of the plant extract composition may be obtained from a first plant and the plant extract of the fibrous supports may be obtained from a second plant.

According to another alternative embodiment, the plant fibers may be obtained from a first plant, the plant extract of the fibrous supports may be obtained from a second plant, and the plant extract of the plant extract composition may be obtained from a third plant.

Advantageously, mixing plants to obtain the reconstituted plant sheet makes it possible to adapt the mechanical properties of the reconstituted plant sheet and/or the organoleptic, chemical or therapeutic properties of the aerosol. Indeed, the fibers of a plant may not have mechanical properties which allow the formation of a fibrous support, but the extract of this plant may confer desired organoleptic properties, chemical properties and/or therapeutic properties on the aerosol. Conversely, the fibers of a plant may have mechanical properties which allow the formation of a fibrous support, but the extract of this plant may not confer the desired organoleptic properties and/or therapeutic properties to the aerosol.

When the plant is the tobacco plant, then the tobacco fibers and the tobacco extract may be obtained from any tobacco plant or tobacco type plant, for example Virginia tobacco, Burley tobacco, air-cured tobacco, dark air-cured tobacco, Oriental tobacco, sun-cured tobacco, fire-cured tobacco, or mixtures thereof.

Typically, the food plants are garlic, coffee, ginger, licorice, rooibos, Stevia rebaudiana, tea, cacao tree, chamomile, mate, star anise, fennel, citronella.

Typically, the aromatic plants are basil, turmeric, clove, laurel, oregano, mint, rosemary, sage, thyme.

Typically, the fragrant plants are lavender, rose, eucalyptus.

Typically, the medicinal plants are those indicated in the document, list A of traditionally used medicinal plants (French pharmacopeia January 2016, published by the Agence Nationale de Sécurité du Médicament (ANSM) [French National Agency for Drug and Health Product Safety]) or plants known to comprise compounds which have therapeutic properties. Typically, the medicinal plants listed are ginkgo, ginseng, sour cherry, peppermint, willow and red vine.

Typically, eucalyptus is among the medicinal plants known to comprise compounds which have therapeutic properties.

Typically, the plant fibers and the plant extract of the reconstituted plant sheet of the present invention may be derived from various plant parts, the plant parts being parts of the plant itself or the result of the processing of various plant parts. Typically, the plant parts may be whole parts of the plant or debris originating from threshing or mixing and shredding the plant parts.

Typically, the plant parts may be selected from the plant parts richest in aromatic compounds conferring on the aerosol its organoleptic properties. Typically, these parts may be the whole plant, the aerial plant parts, such as the flower bud, the branch bark, the stem bark, the leaves, the flower, the fruit and its peduncle, the seed, the petal, the flower head, or the underground parts, for example the bulb, the roots, the root bark, the rhizome, or mixtures thereof. The plant part may also be the result of the mechanical, chemical or mechanical-chemical processing of one or more plant parts, such as for example the shell protecting the cacao bean resulting from the bean dehulling process.

Typically, the tobacco plant parts may be the parts richest in aromatic compounds conferring to the aerosol its organoleptic properties. Typically, the tobacco plant parts may be the parenchyma (lamina) optionally with added stems of the tobacco plant. Typically, the tobacco plant parts may be the leaves of the tobacco plant or the debris originating from threshing or mixing and shredding the leaves and veins of the tobacco plant into shredded tobacco.

Among the food plants, the garlic bulb, the coffee cherry, the star anise fruit, the rhizome of ginger, the licorice root and the leaves of rooibos, Stevia rebaudiana or tea may for example be selected as parts.

Among the aromatic plants, clove flower buds (the cloves), basil, laurel and sage leaves, mint, oregano, rosemary and thyme leaves and flower head, or the turmeric rhizome may for example be selected as parts.

Typically, among the fragrant plants, the lavender flower and flower head, or the rose flower bud and petals may be selected.

Among the medicinal plants listed in the French pharmacopeia, ginkgo leaf, the underground part of ginseng, the peduncle of the sour cherry fruit (cherry stalk), the leaves and flower head of peppermint, the stem bark and the leaves of willow, or the leaves of red vine may for example be selected.

According to one particular embodiment, the reconstituted plant sheet may have a density of between 0.71 g/cm³ and 0.90 g/cm³, a basis weight of between 360 g/m² and 460 g/m² and a thickness of between 480 µm and 520 µm, an S_{AG} of between 19% and 21.5%, an S_{P} of between 37% and 50%, and an S_{P} + S_{AG} of between 58% and 70% and the plant is the tobacco plant.

According to one particular embodiment, the reconstituted plant sheet may comprise:
- a fibrous support comprising refined tobacco fibers and a tobacco extract,
- a fibrous support comprising refined cacao fibers and a cacao extract,
- a tobacco extract composition between the two fibrous supports, and
may have a density comprised between 0.80 g/cm³ and 0.85 g/cm³, a basis weight between 310 g/m² and 320 g/m² and a thickness between 370 µm and 380 µm, an S_{AG} between 16% and 17%, an S_{P} between 40% and 45%, and an S_{P} + S_{AG} comprised between 55% and 65%.

The content by weight of solids of the refined plant fibers included in the reconstituted plant sheet may be comprised between 15% and 50%, in particular between 25% and 45%, more particularly between 30% and 42%.

Typically, the fibrous support of the reconstituted plant sheet may also comprise cellulose-based plant fibers.

Cellulose-based plant fibers are fibers obtained by means of a chemical or mechanical or thermomechanical cooking process, such as wood pulp, hemp, or annual plants such as flax for example. A mixture of these cellulose-based plant fibers may also be used.

Advantageously, these cellulose-based plant fibers may improve the mechanical strength properties of the reconstituted plant sheet.

Typically, the cellulose-based plant fibers may represent between 0.5% and 20%, in particular between 3% and 17.5%, more particularly between 5% and 15% by weight of solids of the reconstituted plant sheet.

According to a second subject, the invention relates to a process for producing a reconstituted plant sheet as defined above, comprising the following steps:
a) passing the refined plant fibers through a papermaking machine so as to produce a base web,
b) bringing a plant extract into contact on the base web to obtain a fibrous support, and
c) bringing a plant extract composition into contact between two fibrous supports so as to obtain the wet reconstituted plant sheet, and
d) drying the wet reconstituted plant sheet to produce the reconstituted plant sheet,
wherein an aerosol-generating agent is incorporated during step b) and/or during step c).

The refined fibers, the base web, the plant extract, the fibrous support, the plant extract composition and the aerosol-generating agent are as described above in relation with the reconstituted plant sheet of the invention.

According to the invention, the base web of the fibrous support is produced using a papermaking process. According to one preferred embodiment of the invention, a reconstituted plant sheet according to the invention is a reconstituted plant sheet that may be obtained by means of a papermaking process.

According to one embodiment, the plant fibers of the fibrous support and the plant extract are obtained according to the following steps:
e) mixing one or more plant parts with a solvent in order to extract the plant extract from the plant fibers,
f) separating the plant extract from the plant fibers.

The plant extract and the plant fibers are therefore typically obtained by means of a dissociation process. During the mixing step e), one or more plant parts are mixed with a solvent, for example in a digester, in order to extract the plant extract from the plant fibers. During the separating step f), the plant extract is separated from the plant fibers, for example by passing through a screw press, in order to isolate and obtain, on the one hand, the plant fibers and, on the other hand, the plant extract.

Typically, the solvent may be an apolar solvent, an aprotic polar solvent, a protic polar solvent, or mixtures thereof, in particular the solvent may be methanol, dichloromethane, ethanol, acetone, butanol, water or mixtures thereof, more particularly the solvent is ethanol, acetone, water or mixtures thereof.

According to one particular embodiment, the solvent is an aqueous solvent, most particularly the solvent is water.

Those skilled in the art will know how to adapt the temperature of the solvent during mixing step e) to the plant, to the plant part and to the plant parts to be treated. Typically, the temperature of the solvent during the treatment of a root or of a bark will be higher than the temperature of the solvent during the treatment of a leaf or a petal.

Typically, the temperature of the solvent during mixing step e) may be between 10°C and 100°C, in particular between 30°C and 90°C, more particularly between 50°C and 80°C.

According to the embodiment in which the solvent is water and the plant is tobacco, the temperature of the water may, for example, be between 30°C and 80°C. Typically, the temperature of the water may be between 50°C and 80°C for the treatment of the parenchyma of a tobacco plant and may be between 30°C and 70°C for the treatment of the stems of a tobacco plant.

The plant fibers may be refined in a refiner and then passed through the papermaking machine so as to produce the base web.

The refined plant fibers may originate from various plants.

The fibers of each plant may be obtained separately according to the dissociation process described above. They may subsequently be mixed such that this mixture of fibers from various plants passes through the papermaking machine so as to produce the base web. It is also possible to obtain fibers from various plants together by bringing together one or more parts of the various plants and then subjecting them to the dissociation process described above. The temperature of the water will then be adapted to the plants to be treated and, in particular, to the plant requiring the highest temperature of the water for extracting the extract of this plant. This alternative embodiment is very advantageous since it makes it possible to obtain the fibers of the various plants without carrying out several dissociation processes in parallel.

Typically, the plant extract may be an extract of various plants.

The extract of various plants may be obtained by mixing various plant extracts obtained separately according to the dissociation process described above. It is also possible to obtain the extract of various plants by bringing together one or more parts of the various plants and then subjecting them to the dissociation process described above. The temperature of the water will then be adapted to the plants to be treated and, in particular, to the plant requiring the highest temperature of the water for extracting the water-soluble extract of this plant. This alternative embodiment is very advantageous since it makes it possible to obtain the extract of various plants without carrying out several processes in parallel. In these two situations, the extract of various plants is brought into contact with the base web during step b).

Typically, various plant extracts, obtained according to the dissociation process described above, may also be brought into contact with the base web separately during step b).

Typically, the plant extract may be concentrated before being brought into contact with the base web during step b). A device such as a vacuum evaporation device may be used to concentrate the plant extract.

When the aerosol-generating agent is incorporated during step b), the plant extract and the aerosol-generating agent may be brought into contact with the base web one after the other, or may be mixed so as to be brought into contact with the base web together.

Typically, step b) of bringing the plant extract into contact may be carried out by impregnation or by spraying, in particular by impregnation. Typically, the impregnation may be carried out by means of a size press.

Step c) of bringing the plant extract composition into contact may be carried out by spraying, by coating, in particular by coating. Typically, the coating may be carried out using a doctor blade, a screw rod, a coating bar or any other item of equipment allowing coating on a support, in particular a threaded rod.

The extract composition used in step c) may be obtained from the plant extract obtained according to the dissociation process described above. Typically, the plant extract may be concentrated before being incorporated into the extract composition. A device such as a vacuum evaporation device may be used to concentrate the plant extract.

When the aerosol-generating agent is incorporated during step c), the aerosol-generating agent may be mixed with the extract composition so that this mixture is brought into contact during step c), the aerosol-generating agent may also be brought into contact with the fibrous support before or after the extract composition.

If the extract composition comprises an additive, then this additive may be mixed with the extract composition so that this mixture is brought into contact during step c), the additive may also be brought into contact with the fibrous support, or the additive and the aerosol-generating agent may be mixed in order to be brought into contact with the fibrous support together.

According to one embodiment, the plant extract composition may be brought into contact during step c) with a first fibrous support and then with a second fibrous support so that the plant extract composition forms a layer between the two fibrous supports so as to obtain the reconstituted plant sheet.

According to this embodiment, step c) may comprise an intermediate drying step between the contacting of the plant extract composition with the first fibrous support and the contacting with the second fibrous support. This intermediate drying step may be carried out using an infrared ramp, American battery drying drums, hot-air drying in a tunnel dryer, a vertical dryer, a fluidized-bed dryer, a pneumatic dryer, in particular in a tunnel dryer. Those skilled in the art will know how to adapt the operating conditions so as to carry out this intermediate drying step.

According to an alternative embodiment, during step c), a first portion of the plant extract composition may be brought into contact with a first fibrous support, a second portion of the plant extract composition may be brought into contact with a second fibrous support, and the two portions of the plant extract composition are brought into contact so as to form a layer between the two fibrous supports in order to obtain the reconstituted plant sheet.

According to this alternative embodiment, step c) may comprise an intermediate drying step between the contacting of the second portion of the plant extract composition with the second fibrous support and the contacting of the two portions of the plant extract composition. This intermediate drying step can be carried out using an infrared ramp, American battery drying drums, hot-air drying in a tunnel dryer, a vertical dryer, a fluidized-bed dryer, a pneumatic dryer, in particular in a tunnel dryer. Those skilled in the art will know how to adapt the operating conditions so as to carry out this intermediate drying step.

S_{P} and S_{AG} of the reconstituted plant sheet produced by the process of the invention may be controlled by controlling the amount of plant extract and the amount of aerosol-generating agent used in steps b) and c). Those skilled in the art will know how to adapt the amount of plant extract and the amount of aerosol-generating agent used in steps b) and c) in order to achieve the target S_{P} and S_{AG}.

Drying step d) may be carried out using an infrared ramp, American battery drying drums, hot-air drying in a tunnel dryer, a vertical dryer, a fluidized-bed dryer, a pneumatic dryer, in particular in a tunnel dryer. Those skilled in the art will know how to adapt the operating conditions so as to carry out drying step d).

According to one embodiment, the fibrous support obtained during step b) may undergo an intermediate drying step between step b) and step c). This intermediate drying step may be carried out using an infrared ramp, American battery drying drums, hot-air drying in a tunnel dryer, a vertical dryer, a fluidized-bed dryer, a pneumatic dryer, in particular in a tunnel dryer. Those skilled in the art will know how to adapt the operating conditions so as to carry out this intermediate drying step.

The reconstituted plant sheet produced by the process for producing a reconstituted plant sheet of the invention may undergo a calendering step.

Thus, one embodiment of the invention is a process for producing a calendered reconstituted plant sheet in which the reconstituted plant sheet produced during drying step d) of the process for producing a reconstituted plant sheet as described above undergoes a calendering step.

For the purposes of the present application, "calendering step" denotes a step consisting in passing the reconstituted plant sheet between two contra-rotating cylinders with adjustable spacing.

Advantageously, the calendering step makes it possible to reduce the thickness of the reconstituted plant sheet without changing the basis weight thereof. This calendering step therefore makes it possible to increase the density of the reconstituted plant sheet in order to achieve, if necessary, the density values of the reconstituted plant sheet.

In addition, the calendering step advantageously makes it possible to improve the cohesion of the two fibrous supports of the reconstituted plant sheet.

The cylinders can apply a pressure to the reconstituted plant sheet of between 1 bar and 250 bar, in particular between 14 bar and 140 bar, very particularly between 25 bar and 40 bar.

Advantageously, carrying out the calendering step with cylinders applying a pressure within the ranges indicated above makes it possible to limit, or even to prevent, deterioration of the reconstituted plant sheet.

The cylinders may be heated to a temperature of less than or equal to 50°C, in particular between 30°C and 50°C, very particularly between 40°C and 45°C.

Advantageously, carrying out the calendering step with cylinders heated to a temperature within the ranges indicated above makes it possible to limit, or even to prevent, deterioration of the reconstituted plant sheet.

The reconstituted plant sheet of the invention may then be cut into sheets, leaves similar to strips of tobacco or rolled into a roll. Several sheets may be assembled in order to form a mixture of sheets.

The reconstituted plant sheet of the invention may be used in a device for heating tobacco without burning it.

Thus, according to a third subject, the invention relates to a consumable for a device for heating tobacco without burning it, comprising a reconstituted plant sheet according to the invention as defined above.

For the purposes of the present invention, the term "consumable" denotes the reconstituted plant sheet according to the invention as such or a container comprising the reconstituted plant sheet according to the invention. The consumable is adapted to be introduced into a device for heating tobacco without burning it, and those skilled in the art will know how to adapt the consumable according to the device for heating tobacco without burning it.

The reconstituted plant sheet according to the invention, as consumable, can be shaped so as to be adapted to the device for heating tobacco without burning it. For example, it may be in particulate form, in the form of crimped sheet, in the form of shredded tobacco, in particular in the form of shredded tobacco having a width of from 0.5 mm to 1 mm.

The container can comprise an external envelope which defines an internal volume wherein the reconstituted plant sheet according to the invention is received. In the internal volume, the reconstituted plant sheet according to the invention may be shaped, in particular in particulate form, in the form of crêped sheet, in the form of shredded tobacco.

Typically, the external envelope can be of cylindrical form, in particular with a circular base.

The external envelope may for example be made of cigarette paper, of cigarette rolling paper, of paper, of plant fiber paper, of metal such as aluminum, in particular of cigarette paper.

As consumable, mention may be made of the reconstituted plant sheet according to the invention in particulate form, the reconstituted plant sheet according to the invention in the form of crêped sheet, the reconstituted plant sheet according to the invention in the form of shredded tobacco, a sachet comprising the reconstituted plant sheet according to the invention, a stick comprising the reconstituted plant sheet according to the invention or a capsule comprising the reconstituted plant sheet according to the invention, in particular the reconstituted plant sheet according to the invention in the form of shredded tobacco or a stick comprising the reconstituted plant sheet according to the invention.

The reconstituted plant sheet according to the invention may, for example, be in particulate form in a sachet or in a capsule. The reconstituted plant sheet according to the invention may in particular be in the form of shredded tobacco in a stick.

According to one embodiment, the reconstituted plant sheet included in the consumable may be able to be obtained by the process according to the invention as defined above.

Typically, the ratio between the mass of reconstituted plant sheet of the invention and the internal volume of the consumable may be, for example, of between 300 mg/ml and 500 mg/ml, in particular between 355 mg/ml and 450 mg/ml, more particularly between 375 mg/ml and 425 mg/ml.

Advantageously, a ratio within these ranges of values makes it possible to generate an aerosol having satisfactory organoleptic properties. In particular, the aerosol does not have very pronounced organoleptic properties when the ratio is less than these ranges of values. In addition, when the ratio is greater than these ranges of values, the draw resistance is too great for smoking to be satisfactory.

According to a fourth subject, the invention relates to the use of a reconstituted plant sheet according to the invention as defined above or of a consumable according to the invention as defined above in a heating device, in particular a device for heating tobacco without burning it.

Typically, the sheet may be used alone or in a mixture with a natural plant leaf, a conventional reconstituted plant sheet, in particular a natural tobacco leaf or a conventional reconstituted tobacco sheet.

For the purposes of the present application, the term "device for heating tobacco without burning it" denotes any device which allows the formation of an aerosol intended to be inhaled by a consumer. The aerosol replaces the smoke, thus allowing the user to inhale the plant aromas while at the same time very significantly reducing the user's exposure to the harmful constituents.

Typically, a heating device comprises, in the direction of the air flow, an air inlet, a heating body, a lodging intended to put in place and hold the reconstituted plant sheet of the invention comprising the aerosol-generating agent, and an air outlet intended to be introduced into the mouth of the user. The air inlet, the heating body, the lodging and the air outlet are typically connected at least fluidically to one another.

Typically, when the heating device is used, air is sucked into the heating device via the air inlet by the user; the air sucked in then passes through the heated portion so as to obtain heated air; on contact with the reconstituted plant sheet of the invention comprising the aerosol-generating agent, held in the lodging, an aerosol is formed by the heated air and is then inhaled by the user. If the plant is a medicinal plant, then the aerosol formed has therapeutic properties.

Furthermore, by virtue of the heating device, there is no combustion of the sheet. The user may therefore take advantage of the organoleptic properties of the plant, and optionally of the tobacco, while at the same time very significantly reducing their exposure to the harmful constituents.

### Examples

### Example 1.1: Calendered reconstituted tobacco sheet according to the invention

A mixture of scraps and stems of tobacco of Virginia type is brought into contact with water at 65°C with stirring for 45 minutes. The tobacco extract is separated from the fibrous portion by mechanical pressing. The tobacco extract is concentrated under vacuum to a solids concentration of 60%.
The tobacco fibers are refined and then passed through a laboratory papermaking machine to produce two base webs having a basis weight of approximately 86 g/m².
Glycerol, as aerosol-generating agent, is added to the concentrated tobacco extract. The concentrated tobacco extract comprising the glycerol is brought into contact with each of the two base webs by impregnation in a size press in order to produce two fibrous supports which are then dried on a laboratory drying plate.

A tobacco extract composition is produced from the concentrated extract and the glycerol. The tobacco extract composition is spread over a first fibrous support and then a second fibrous support is placed onto the tobacco extract composition so that the tobacco extract composition forms a layer between the two supports and that a reconstituted tobacco sheet is then produced after having been dried on a laboratory drying plate.

The reconstituted tobacco sheet undergoes a step of calendering using cylinders applying a pressure of 34.5 bar to produce the calendered reconstituted tobacco sheet which is then dried on a laboratory drying plate.

The contents of tobacco extract and of glycerol are adjusted such that the calendered reconstituted tobacco sheet has an S_{P} of 37.1%, an S_{AG} of 21.2% and a sum total S_{AG} + S_{P} of 58.3%.

The calendered reconstituted tobacco sheet has a density of 0.71 g/cm³, a basis weight of 368 g/m² and a thickness of 515 µm.

### Example 1.2: Calendered reconstituted plant sheet in according to the invention comprising a fibrous support comprising refined tobacco fibers, a fibrous support comprising refined cacao fibers, and a tobacco extract composition.

The fibrous support comprising refined tobacco fibers and the tobacco extract composition are produced according to the method described above.

Cacao shells are ground using a knife mill in order to obtain particles of the order of 1 mm. The ground shell material is then mixed with water at 70°C for 20 minutes at a shell/water ratio of 1/10. The mixture is then centrifuged in order to separate the cacao tree extract from the cacao fibers and to recover firstly the cacao tree extract and secondly the cacao shell fibers. The cacao fibers are refined and then passed through a laboratory papermaking machine to produce a cacao base web having a basis weight of approximately 60 g/m².
The cacao tree extract is concentrated under vacuum to a solids concentration of 19.5%.
Glycerol, as aerosol-generating agent, is added to the concentrated cacao tree extract. The concentrated cacao extract comprising glycerol is brought into contact with the cacao base web by impregnation in a size press in order to produce the fibrous support comprising refined cacao fibers.

A portion of the tobacco extract composition is then spread over the fibrous support comprising refined tobacco fibers, and the other portion of the tobacco extract composition is spread over the fibrous support comprising refined cacao fibers. The two fibrous supports are stacked one on top of the other such that the two portions of tobacco extract composition form a layer between the fibrous supports. A reconstituted tobacco sheet is thus produced and undergoes the calendering step described above.

The contents of tobacco extract, of cacao tree extract and of glycerol are adjusted in order to obtain a reconstituted plant sheet produced having an S_{P} of 43.6%, with 40.9% tobacco and 2.7% cacao tree, an S_{AG} of 16.3% and a sum total S_{AG} + S_{P} of 59.9%.

This reconstituted plant sheet has a density of 0.83 g/cm³, a basis weight of 312 g/m² and a thickness of 376 µm.

### Comparative example 1: Reconstituted tobacco sheet not in accordance with the invention

The reconstituted tobacco sheet not in accordance with the invention comprises a single fibrous support produced according to a mode of operation similar to that described above.

The contents of tobacco extract and of glycerol are adjusted such that the reconstituted tobacco sheet not in accordance with the invention has an S_{P} of 29.4%, an S_{AG} of 19% and a sum total S_{AG} + S_{P} of 48.4%.

The reconstituted tobacco sheet not in accordance with the invention has a density of 0.58 g/cm³, a basis weight of 151 g/m² and a thickness of 261 µm.

The density of this sheet is less than 0.6 and is lower than the density of the sheets of Examples 1.1 to 1.2.

### Example 2: Comparisons of the pressure drop and organoleptic properties of the aerosols generated by the sheets of example 1.1 and comparative example 1

Various sticks are tested in this example 2. The stick comprising the sheets of example 1.1 is denoted stick of example 1.1. The sticks comprising the sheets of comparative example 1 are denoted sticks of comparative example 1. The reconstituted tobacco sheets in accordance with the invention and a tobacco sheet not in accordance with the invention are cut up into shredded tobacco, and then introduced at a given mass into Neo Navy Blue stick sheaths for glo^{™} in order to obtain the desired ratio.

The pressure drop is a parameter which makes it possible to characterize the draw resistance of a stick. Specifically, the greater the pressure drop across this stick, the greater the draw resistance of the stick. The pressure drop is determined according to the following protocol. An SMI PDV SODIM instrument is used, this being an apparatus for measuring the draw resistance which measures the difference in pressure drop at a constant air flow of 17.5 ml/s during the measurement through the stick containing the reconstituted plant sheet. The draw resistance is expressed in millimeters of water column (mm WC).

The organoleptic properties of the aerosols generated by the various sticks tested are evaluated by an independent expert according to the following protocol. The tobacco sheet in accordance with the invention and a tobacco sheet not in accordance with the invention are evaluated in succession by an independent expert in a glo^{™} type heating system, the taste of the aerosol is graded out of 10.

The results are given in table 1 below.

**[Table 1]**

| Stick of example 1.1 | | | Sticks of comparative example 1 | | |
|---|---|---|---|---|---|
| Ratio (mg/ml) | Pressure drop (mm WC) | Taste | Ratio (mg/ml) | Pressure drop (mm WC) | Taste |
| | | | 297 | 77 | Not satisfactory |
| 400 | 76 | Very satisfactory | 400 | 86 | Not satisfactory |

The results of table 1 demonstrate that, for a given ratio of 400 mg/ml, the pressure drop:
- of the stick of example 1.1 is 76, which is acceptable, and
- of the stick of comparative example 1 is 86, which is a value which is too high for the smoking of the stick of comparative example 1 to be satisfactory.

The ratio is then reduced to 297 so that the pressure drop of the stick of comparative example 1 is of the same order of magnitude as that of the stick of example 1.1. The results of table 1 demonstrate that, for a pressure drop of the same order of magnitude (76 and 77), the aerosol generated by:
- the stick of example 1.1 has very satisfactory organoleptic properties (grade of 8/10), and in particular the aerosol has a pronounced Virginia tobacco taste, an advantageous amplitude, a good, round and balanced taste which is superior to the aerosols formed when smoking the tobacco sheet not in accordance with the invention, and
- the stick of comparative example 1 has unsatisfactory organoleptic properties (grade of 2/10), especially a tobacco taste which is not very pronounced.

This example 2 therefore demonstrates that the reconstituted plant sheet according to the invention makes it possible to obtain a good compromise between the draw resistance and the organoleptic properties of the aerosol.

## Claims

1. Reconstituted plant sheet comprising:
- two fibrous supports, each fibrous support comprising refined plant fibers and a plant extract,
- a plant extract composition between the two fibrous supports, and
- an aerosol-generating agent,
**characterized in that** the density of the reconstituted plant sheet is greater than or equal to 0.60 g/cm³.

2. Reconstituted plant sheet according to Claim 1, the density of which is between 0.62 g/cm³ and 1.50 g/cm³.

3. Reconstituted plant sheet according to Claim 1 or Claim 2, wherein the plant is the tobacco plant.

4. Reconstituted plant sheet according to any one of Claims 1 to 3, wherein the total content by weight of solids of aerosol-generating agent is between 10% and 30%.

5. Reconstituted plant sheet according to any one of Claims 1 to 4, wherein the total content by weight of solids of the plant extract is between 30% and 60%.

6. Reconstituted plant sheet according to any one of Claims 1 to 5, wherein the sum of the total content by weight of solids of plant extract and the total content by weight of solids of aerosol-generating agent is between 40% and 80%.

7. Reconstituted plant sheet according to any one of Claims 1 to 6, the thickness of which is between 250 µm and 1050 µm.

8. Reconstituted plant sheet according to any one of Claims 1 to 7, the basis weight of which is greater than 200 g/m².

9. Reconstituted plant sheet according to any one of Claims 1 to 8, wherein the aerosol-generating agent is chosen from sorbitol, glycerol, propylene glycol, triethylene glycol, erythritol, propanediol, lactic acid, benzyl benzoate, glyceryl diacetate, glyceryl triacetate, triethyl citrate, isopropyl myristate, and mixtures thereof.

10. Reconstituted plant sheet according to any one of Claims 1 to 9 which has been calendered.

11. Process for producing a reconstituted plant sheet as defined in Claims 1 to 9, comprising the following steps:
a) passing the refined plant fibers through a papermaking machine so as to produce a base web,
b) bringing a plant extract into contact on the base web to obtain a fibrous support, and
c) bringing a plant extract composition into contact between two fibrous supports so as to obtain the wet reconstituted plant sheet, and
d) drying the wet reconstituted plant sheet to produce the reconstituted plant sheet,
wherein an aerosol-generating agent is incorporated during step b) and/or during step c).

12. Process for producing a reconstituted plant sheet as defined in Claim 10, wherein the reconstituted plant sheet produced during drying step d) of the process for producing a reconstituted plant sheet as defined in Claim 11 undergoes a calendering step.

13. Consumable for a device for heating tobacco without burning it, comprising a reconstituted plant sheet as defined in any one of Claims 1 to 10.

14. Consumable as defined in claim 13 being a sachet comprising the reconstituted plant sheet, a stick comprising the reconstituted plant sheet or a capsule comprising the reconstituted plant sheet.

15. Non-therapeutic use of a reconstituted plant sheet as defined in any one of Claims 1 to 10 in a heating device.

16. Reconstituted plant sheet as defined in any one of Claims 1 to 10 for use thereof in a heating device for therapeutic purposes, the plant being chosen from medicinal plants.

## Patentansprüche

1. Rekonstituiertes Pflanzenblatt, umfassend:
- zwei faserige Träger, wobei jeder faserige Träger veredelte Pflanzenfasern und einen Pflanzenextrakt umfasst,
- eine Pflanzenextrakt-Zusammensetzung zwischen den beiden faserigen Trägern, und
- ein aerosolerzeugendes Mittel,
**dadurch gekennzeichnet, dass** die Dichte des rekonstituierten Pflanzenblattes größer als oder gleich 0,60 g/cm³ ist.

2. Rekonstituiertes Pflanzenblatt nach Anspruch 1, dessen Dichte zwischen 0,62 g/cm³ und 1,50 g/cm³ liegt.

3. Rekonstituiertes Pflanzenblatt nach Anspruch 1 oder Anspruch 2, wobei die Pflanze die Tabakpflanze ist.

4. Rekonstituiertes Pflanzenblatt nach einem der Ansprüche 1 bis 3, wobei der Gesamtgewichtsgehalt an Feststoffen eines aerosolerzeugenden Mittels zwischen 10% und 30% liegt.

5. Rekonstituiertes Pflanzenblatt nach einem der Ansprüche 1 bis 4, wobei der Gesamtgewichtsgehalt an Feststoffen des Pflanzenextrakts zwischen 30% und 60% liegt.

6. Rekonstituiertes Pflanzenblatt nach einem der Ansprüche 1 bis 5, wobei die Summe des Gesamtgewichtsgehalt an Feststoffen eines Pflanzenextrakts und des Gesamtgewichtsgehalts an Feststoffen eines aerosolerzeugenden Mittels zwischen 40% und 80% liegt.

7. Rekonstituiertes Pflanzenblatt nach einem der Ansprüche 1 bis 6, dessen Dicke zwischen 250 µm und 1050 µm liegt.

8. Rekonstituiertes Pflanzenblatt nach einem der Ansprüche 1 bis 7, dessen Flächengewicht größer als 200 g/m² ist.

9. Rekonstituiertes Pflanzenblatt nach einem der Ansprüche 1 bis 8, wobei das aerosolerzeugende Mittel ausgewählt ist aus Sorbit, Glycerin, Propylenglykol, Triethylenglykol, Erythrit, Propandiol, Milchsäure, Benzylbenzoat, Glyceryldiacetat, Glyceryltriacetat, Triethylcitrat, Isopropylmyristat, und Mischungen davon.

10. Rekonstituiertes Pflanzenblatt nach einem der Ansprüche 1 bis 9, das kalandriert worden ist.

11. Verfahren zur Herstellung eines rekonstituierten Pflanzenblattes wie in den Ansprüchen 1 bis 9 definiert, umfassend die folgenden Schritte:
a) Durchleiten der veredelten Pflanzenfasern durch eine Papierherstellungsmaschine zur Herstellung einer Rohbahn,
b) Inkontaktbringen eines Pflanzenextraktes mit der Rohbahn, um einen faserigen Träger zu erhalten, und
c) Inkontaktbringen einer Pflanzenextraktzusammensetzung zwischen zwei faserigen Trägern, um das nasse rekonstituierte Pflanzenblatt zu erhalten, und
d) Trocknen des nassen rekonstituierten Pflanzenblattes, um das rekonstituierte Pflanzenblatt herzustellen,
wobei ein aerosolerzeugendes Mittel während Schritt b) und/oder während Schritt c) inkorporiert wird.

12. Verfahren zur Herstellung eines rekonstituierten Pflanzenblattes wie in Anspruch 10 definiert, wobei das rekonstituierte Pflanzenblatt, das während des Trocknungsschritts d) des Verfahrens zur Herstellung eines rekonstituierten Pflanzenblattes wie in Anspruch 11 definiert, einem Kalandrierungsschritt unterzogen wird.

13. Verbrauchsmaterial für eine Vorrichtung zum Erhitzen von Tabak, ohne diesen zu verbrennen, umfassend ein rekonstituiertes Pflanzenblatt wie in einem der Ansprüche 1 bis 10 definiert.

14. Verbrauchsmaterial wie in Anspruch 13 definiert, das ein Beutel, umfassend das rekonstituierte Pflanzenblatt, ein Stick, umfassend das rekonstituierte Pflanzenblatt, oder eine Kapsel, umfassend das rekonstituierte Pflanzenblatt, ist

15. Nicht-therapeutische Verwendung eines rekonstituierten Pflanzenblattes wie in einem der Ansprüche 1 bis 10 definiert in einer Heizvorrichtung.

16. Rekonstituiertes Pflanzenblatt wie in einem der Ansprüche 1 bis 10 definiert zur Verwendung in einer Heizvorrichtung für therapeutische Zwecke, wobei die Pflanze aus Heilpflanzen ausgewählt ist.

## Revendications

1. Feuille de plante reconstituée comprenant :
- deux supports fibreux, chaque support fibreux comprenant des fibres végétales raffinées et un extrait de plante,
- une composition d'extrait de plante entre les deux supports fibreux, et
- un agent générateur d'aérosol,
**caractérisée en ce que** la densité de la feuille de plante reconstituée est supérieure ou égale à 0,60 g/cm³.

2. Feuille de plante reconstituée selon la revendication 1, dont la densité est comprise entre 0,62 g/cm³ et 1,50 g/cm³.

3. Feuille de plante reconstituée selon la revendication 1 ou la revendication 2, dans laquelle la plante est le plan de tabac.

4. Feuille de plante reconstituée selon l'une quelconque des revendications 1 à 3, dans laquelle la teneur totale en solides d'agent générateur d'aérosol est comprise entre 10 % et 30 %.

5. Feuille de plante reconstituée selon l'une quelconque des revendications 1 à 4, dans laquelle la teneur totale en solides de l'extrait de plante est comprise entre 30 % et 60%.

6. Feuille de plante reconstituée selon l'une quelconque des revendications 1 à 5, dans laquelle la somme de la teneur totale en solides d'extrait de plane et de la teneur totale en solides de l'agent générateur d'aérosol est comprise entre 40 % et 80 %.

7. Feuille de plante reconstituée selon l'une quelconque des revendications 1 à 6, dont l'épaisseur est comprise entre 250 µm et 1050 µm.

8. Feuille de plante reconstituée selon l'une quelconque des revendications 1 à 7, dont le poids de base est supérieur à 200 g/m².

9. Feuille de plante reconstituée selon l'une quelconque des revendications 1 à 8, dans laquelle l'agent générateur d'aérosol est choisi parmi le sorbitol, le glycérol, le propylène, le glycol, le triéthylène glycol, l'érythritol, le propanediol, l'acide lactique, le benzoate de benzyle, le diacétate de glycéryle, le triacétate de glycéryle, le citrate de triéthyle, le myristate d'isopropyle et des mélanges de ceux-ci.

10. Feuille de plante reconstituée selon l'une quelconque des revendications 1 à 9, qui a été calandrée.

11. Procédé de fabrication d'une feuille de plante reconstituée selon les revendications 1 à 9, comprenant les étapes suivantes :
a) passage des fibres de plantes raffinées à travers une machine à papier de manière à produire une feuille de base,
b) mise en contact d'un extrait de plante en contact sur la feuille de base pour obtenir un support fibreux, et
c) mise en contact d'une composition d'extrait de plante en contact entre deux supports fibreux de manière à obtenir la feuille de plante reconstituée humide, et
d) séchage de la feuille de plante reconstituée humide pour fabriquer la feuille de plante reconstituée,
dans lequel un agent générateur d'aérosol est incorporé au cours de l'étape b) et/ou au cours de l'étape c).

12. Procédé de fabrication d'une feuille de plante reconstituée selon la revendication 10, dans lequel la feuille de plante reconstituée fabriquée au cours de l'étape de séchage d) du procédé de fabrication d'une feuille de plante reconstituée selon la revendication 11 est soumise à une étape de calandrage.

13. Consommable pour un dispositif de chauffage de tabac sans le brûler, comprenant une feuille de plante reconstituée selon l'une quelconque des revendications 1 à 10.

14. Consommable tels que définie dans la revendication 13 étant un sachet comprenant la feuille de plante reconstituée, un stick comprenant la feuille de plante reconstituée ou une capsule comprenant la feuille de plante reconstituée.

15. Utilisation non thérapeutique d'une feuille de plante reconstituée selon l'une quelconque des revendications 1 à 10 dans un dispositif de chauffage.

16. Feuille de plante reconstituée selon l'une quelconque des revendications 1 à 10 destinée à une utilisation dans un dispositif de chauffage à des fins thérapeutiques, la plante étant choisie parmi des plantes médicinales.
